# EUROPEAN PATENT APPLICATION

(11) **EP 1 852 498 A1**
(43) Date of publication of application: **07.11.2007**
(21) Application number: 06702022.2
(22) Date of filing: 04.01.2006
(51) Int. Cl.: C12N 1/20, A23L 1/30, A23L 2/52, A61K 35/74, A61P 37/08, C12R 1/23

(54) **METHOD OF PRODUCING LACTIC ACID BACTERIUM HAVING ANTIALLERGIC EFFECT**

(30) Priority: 04.01.2005 JP 2005024204
(71) Applicant: Calpis Co., Ltd., Shibuya-ku, Tokyo 150-0022 (JP)
(72) Inventor: FUJIWARA, Shigeru, Sagamihara-shi, Kanagawa 229-0001 (JP); SAWADA, Daisuke, Machida-shi, Tokyo 194-0045 (JP); YOSHIKAWA, Akira, Nagoya-shi, Aichi, 463-0032 (JP); MIZUTANI, Jun, Kanagawa, 220-0104 (JP)
(74) Representative: Vossius & Partner
(86) International application number: PCT/JP2006/300004
(87) International publication number: WO 2006/073145

(57) **Abstract**

Disclosed is a method for preparing lactic acid bacteria having a high anti-allergic activity comprising culturing the lactic acid bacteria in a medium containing casein hydrolysate. The invention also provides an anti-allergic agent and a food/beverage product having anti-allergic activity comprising the lactic acid bacteria cultured in the method of the invention.

## Description

### TECHNICAL FIELD

The present invention relates to a method for the preparation of lactic acid bacteria which enhance the productivity of cytokine in a living body upon oral ingestion and exhibit an excellent anti-allergic activity. The present invention also relates to an anti-allergic agent and a food/beverage product containing the lactic acid bacteria as an effective ingredient.

### BACKGROUND ART

Lactic acid bacteria have been known to have an effect of improving balance of intestinal flora to decrease the putrefied products in intestine and to improve the properties of feces, as well as an effect of enhancing the immune activity of an organism. Lactic acid bacteria have been utilized as an effective ingredient of an anti-allergic agent (see, for example, Japanese Patent Laid-Open No. 2004-026729 [Patent Document 1]. It has been generally said that relatively large amounts of the cells have to be ingested in order to achieve the physiological effect by lactic acid bacteria.

Lactic acid bacteria are conventionally cultured in a commercially available research-grade MRS medium. Nutrients such as yeast extract and beef extract are used as additives for enhancing the growth of lactic acid bacteria.

It is obvious that lactic acid bacteria will grow normally in a medium which satisfis the requirements for nutrition of lactic acid bacteria. However, when a medium satisfying the requirements for nutrition of lactic acid bacteria as described above is used for culturing lactic acid bacteria in large quantity in an industrial scale, there is a problem that a large amount of cells having an anti-allergic activity is not always produced at a low cost.

In addition, since the MRS medium is designed for research use, it is not desirable to include lactic acid bacteria cultured in this medium in food and beverage products. Thus, there has been a demand for developing a medium which solely comprises those components which are approved for use in food and food additives.

It has also been known that materials such as peptone (a partially hydrolyzed protein which is not precipitated by ammonium sulfate; secondary protein derivative used as a component in a medium for culturing microorganisms) may be used as one of the nutritional sources for the medium. However, it has not been known at all whether the type of peptone contained in the medium is related to the anti-allergic activity of lactic acid bacteria grown in that medium. Particularly, it has not been known at all that a sufficient level of anti-allergic activity cannot be achieved in some cases, even if the number of the cells of lactic acid bacteria increases.

No research has been reported so far with regard to the correlation between the anti-allergic activity of lactic acid bacteria and the composition of the medium used for culturing the bacteria. At present, the composition of the medium is selected only on the growth characteristics of the lactic acid bacteria.

An object of the present invention is to provide a method for preparing lactic acid bacteria with a high anti-allergic activity by selecting the medium composition which is applicable to food/beverage production, as well as an anti-allergic agent comprising the lactic acid bacteria prepared by the method as an effective ingredient, and food/beverage products having an anti-allergic activity comprising the lactic acid bacteria as an effective ingredient.

### DISCLOSURE OF THE INVENTION

The inventors have carried out intensive studies for achieving the above objects and found that lactic acid bacteria having a high anti-allergic activity can be produced by culturing the bacteria in a medium containing casein hydrolysate (casein peptone).

In one aspect, the present invention provides a method for preparing a lactic acid bacterium having an anti-allergic activity comprising culturing the lactic acid bacterium in a medium containing casein hydrolysate in an amount of not less than 0.01% by mass, preferably from 0.01 to 10% by mass.

According to the present invention, lactic acid bacteria having a high anti-allergic activity can be obtained by culturing the lactic acid bacteria in a medium containing casein hydrolysate. Further, a larger amount of lactic acid bacteria can be be produced by using a medium containing nitrogen sources derived from farmed meat or fish meat besides the casein hydrolysate.

The medium preferably contains 0.01 to 10% by mass of casein hydrolysate, whereby lactic acid bacteria having higher anti-allergic activity may be obtained. Also preferably, a medium containing from 0.01 to 10% by of nitrogen sources derived from farmed meat or fish meat for the purpose of promoting the growth of the cells. The nitrogen source derived from farmed meat or fish meat includes, for example, nitrogen sources derived from cattle, tuna, and bonito.

It is also preferred that the lactic acid bacterium used in the invention is selected from *Lactobacillus acidophilus, Lactobacillus fermentum, Lactobacillus casei, Lactobacillus paracasei* and *Lactobacillus gasseri. Lactobacillus acidophilus* L-92 strain (FERM BP-4981) is particularly preferred. Also preferably, cells of the lactic acid bacteria are collected from the culture and dried to obtain lactic acid bacteria which have a high anti-allergic activity and provide improved safety as a material for food and pharmaceuticals. Accordingly, it is possible to prepare an anti-allergic agent and food/beverage products having a high anti-allergic activity comprising the lactic acid bacteria produced by the method of the invention.

According to the present invention, lactic acid bacteria having a high anti-allergic activity may be produced in larger amount by culturing the lactic acid bacteria in a medium containing casein hydrolysate.

The present invention provides a method for preparing a lactic acid bacterium having an anti-allergic activity, comprising culturing the lactic acid bacterium in a medium containing casein hydrolysate.

In a preferred embodiment, the casein hydrolysate is pancreatin hydrolysate, also preferably the hydrolysate is peptone. In another embodiment, the medium of the invention contains between 0.01 and 10% by mass of casein hydrolysate.

In another embodiment, the lactic acid bacterium is a lactic acid bacterium belonging to genus *Lactobacillus.* Preferably, the lactic acid bacterium belonging to genus *Lactobacillus* is of *Lactobacillus acidophilus, Lactobacillus fermentum, Lactobacillus casei, Lactobacillus* paracasei or *Lactobacillus gasseri.* More preferably, the bacterium of *Lactobacillus acidophilus* is of *Lactobacillus acidophilus* L-92 strain (FERM BP-4981).

In another embodiment, the cells of the lactic acid bacterium is collected from the culture and dried.

In another embodiment, the medium contains casein hydrolysate and a nitrogen source derived from farmed meat or fish meat.

The present invention also provides an anti-allergic agent comprising as an effective ingredient the lactic acid bacterium prepared by the method of the present invention.

The present invention also provides a food/beverage product having an anti-allergic activity comprising the lactic acid bacterium prepared by the method of the invention as an effective ingredient.

Incidentally, the method for preparing lactic acid bacteria having an anti-allergic activity in accordance with the present invention is also able to be used as a method for preparing an anti-allergic agent. Also the culture of the lactic acid bacteria produced by the method of the invention is also able to be used an anti-allergic agent.

In accordance with the method of the invention, lactic acid bacteria having a great activity of stimulating the production of IL-12 may be obtained. Thus, the method for preparing lactic acid bacteria having an anti-allergic activity is also able to be used as a method for preparing an agent for stimulating production of IL-12 comprising the lactic acid bacteria as an effective ingredient. Further, the anti-allergic agent of the present invention is also able to be used as an agent for stimulating production of IL-12 comprising the lactic acid bacteria as an effective ingredient.

In accordance with the present invention, lactic acid bacteria are cultured in a medium containing casein hydrolysate and optionally containing nitrogen source derived from farmed meat or fish meat so that the lactic acid bacteria having an excellent anti-allergic activity may be obtained. In addition, the medium can be prepared at a very low cost and can effectively promote the growth of lactic acid bacteria, whereby the cost for culturing lactic acid bacteria can be reduced. Moreover, it is possible to provide an anti-allergic agent and food/beverage products with improved safety and high anti-allergic effect by using the lactic acid bacteria prepared by the method of the invention as an effective ingredient.

### BRIEF DESCRIPTION OF THE DRAWINGS

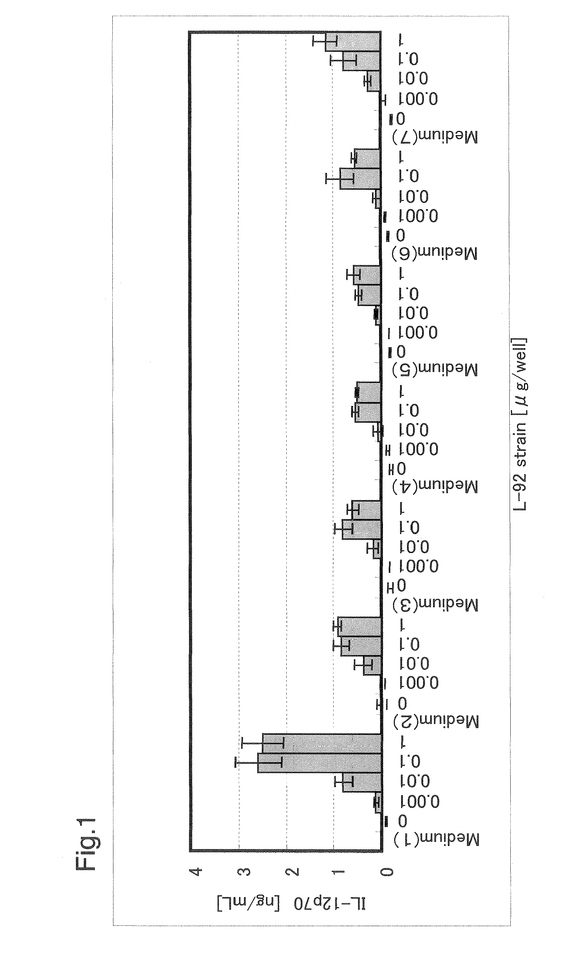

Fig. 1 shows the amount of IL-12 produced from the lactic acid bacteria cultured in the media (1) to (7) of Example 1.

### DETAILED DESCRIPTION OF THE PREFERRED EMBODIMENTS

First, a medium of the present invention used for culturing the lactic acid bacteria will be illustrated. Casein hydrolysate has been known in general as casein peptone or milk peptide, and contains a large amount of nitrogen compounds derived from casein, such as peptides and amino acids. Casein hydrolysate has been commonly used as a food material and a component of culture media. Any of the commercially available products may be used in the present invention.

The nitrogen source derived from farmed meat or fish meat used for the purpose of promoting the growth of cells in the invention includes concentrated broth obtained in the manufacture of fishery/stockbreeding canned products and fishery/stockbreeding processed products, which contains nitrogen compounds such as proteins, peptides and amino acids and inorganic components solved out from farmed meat and fish meat, and which is less expensive. As a nitrogen source derived from farmed meat and fish meat, any of the commercially available products may be used, or prepared by concentrating the extract of farmed meat or fish meat or by enzymatic degradation or acid hydrolysis. Preferred farmed meat is beef and preferred fish meat is tuna and bonito.

Any types of casein hydrolysate may be used in the invention so far as it is a hygienic and safe material suitable for use in the manufacture of food/beverage products, including, for example, "Bacto Casitone" (trade name), "Bactotryptone" (trade name) (both manufactured by Difco), "Paticase Peptone" (trade name) (manufactured by Kyokuto Seiyaku) and "Casein Peptone Plus" (manufactured by Organotechnie).

With regard to the nitrogen source derived from farmed meat and fish meat used for the purpose of promoting the growth of cells, any types of nitrogen source may be used so far as it is a hygienic and safe material suitable for use in the manufacture of food, including, for example, "Bacto Beef Extract" (trade name) (manufactured by Difco), "Bacterio-N-KS(B)" (trade name) (manufactured by Maruha) and "Cultivator T" (trade name) (manufactured by Yaizu Suisan).

In the present invention, it is preferred to use a medium containing not less than 0.01% by mass of casein hydrolysate or, preferably, 0.01 to 10% by mass of casein hydrolysate. If the amount of casein hydrolysate is too small, good growth of lactic acid bacteria cannot be expected, and it is not possible to prepare a large amount of lactic acid bacteria having a sufficient anti-allergic activity, that is an IL-12 inducting ability.

More preferably, a medium contains 0.01 to 10% by mass of nitrogen source derived from farmed meat or fish meat, even more preferably 0.01 to 1% by mass of nitrogen source derived from farmed meat or fish meat, whereby effective growth of cells having an anti-allergic activity may be achieved.

Besides the basic components as described above, it is preferred to add to the medium a saccharide source such as lactose, glucose, sucrose and dextrin, organic or inorganic salt, nitrogen source derived from proteins (including a hydrolysate thereof) other than farmed meat, fish meat and casein. Among them, it is preferred to add 1 to 10% by mass of a saccharide source and 0.5 to 2% by mass of an organic or inorganic salt. If necessary, yeast extract and other types of peptone and meat extract may be also added.

The medium used in the present invention may be prepared according to a conventional method using the components described above. For example, predetermined amounts of farmed meat or fish meat and casein hydrolysate are added to water (preferably, distilled water or deionized water), and optionally other components are added, then the mixture is homogeneously dissolved, adjusted to pH 6.5-7.0, and sterilized by a known method (such as by autoclave).

In the method of the present invention, lactic acid bacteria are cultured according to a conventional method using the medium prepared by the manner described above. For example, a pre-culture of lactic acid bacteria (or colony of lactic acid bacteria grown on an agar medium) is inoculated into the medium and cultured at 30 to 45°C for 12 to 72 hours under aerobic, sub-aerobic or anaerobic conditions. Incidentally, as the pH of the medium will decrease during the culture due to metabolite (such as lactic acid) of lactic acid bacteria, the pH of the medium is monitored and an aqueous alkali solution (aqueous solution of sodium hydroxide, potassium hydroxide, calcium hydroxide or ammonia) is added so as to keep the pH of the medium at 6.5-7.5 (more preferably, 6.8-7.2). In some cases, the pH of the medium is kept at nearly neutral range to keep good conditions for lactic acid bacteria to grow and achieve enhanced yield of the bacterial cells.

Preferred examples of lactic acid bacteria used in the culture method of the present invention include *Lactobacillus delbrueckii bulgaricus, Lactobacillus delbrueckii lactis, Lactobacillus helveticus, Lactobacillus acidophilus, Lactobacillus crispatus, Lactobacillus amylovorus, Lactobacillus gallinarum, Lactobacillus gasseri, Lactobacillus johnsonii, Lactobacillus casei, Lactobacillus paracasei, Lactobacillus zeae, Lactobacillus plantarum, Lactobacillus rhamonosus, Lactobacillus brevis, Lactobacillus kefir, Lactobacillus fermentum, Lactobacillus reuteri, Bifidobacterium longum, Bifidobacterium lactis* and *Bifidobacterium catenulatum.* Particularly preferred are *Lactobacillus acidophilus* L-0062 strain (FERM BP-4980) and L-92 strain (FERM BP-4981), and *Lactobacillus fermentum* CP34 strain (FERM BP-8383), which will provide higher anti-allergic activity.

After completion of culturing lactic acid bacteria, the cells of the lactic acid bacteria collected by, for example, filtration or centrifugation from the culture medium, and the cells are dried to provide lactic acid bacteria having higher stability suitable as a material for food/beverage products and pharmaceuticals. In addition, the cells maintain its anti-allergic activity even after the cells are dead upon sterilization by heating before drying. It is preferred that dried cells are pulverized and made into a material for food/beverage products and pharmaceuticals.

The anti-allergic agent of the present invention contains the lactic acid bacteria as an effective ingredient. Such an anti-allergic agent may be prepared by addition of excipient, sweetener, acidic material, vitamins, minerals, dye, fragrance, thickening polysaccharide, if necessary, to the lactic acid bacteria to form powder, granules, tablets, capsules, paste, jelly or various kinds of solid food or liquid beverage.

The anti-allergic agent of the present invention has an immunostimulating effect and may be used as an immunostimulator. Its effective dose for adult is between 1 mg and 10 g per day, more preferably between 10 and 200 mg per day of the lactic acid bacteria.

The anti-allergic agent of the present invention may also be ingested in the form of beverage, jelly, candy, chewing gum, retort pouch or instant food. In that case, it is preferred that the lactic acid bacteria are added to the food to be ingested in an amount of between 1 and 1,000 mg per day, more preferably between 10 and 500 mg per day.

The disclosure of all patens and documents cited herein are entirely incorporated herein as reference. The present application claims priority based on Japanese Patent Application No. 2005 - 24204, the disclosure of which is entirely incorportaed herein as reference.

The present invention will now be specifically illustrated by way of the following Examples.

### EXAMPLES

### Example 1

### Preparation of a medium containing casein peptone extract

Casein peptone (trade name: Casein Peptone Plus; manufactured by Organotechnie) (10 g) was added to 10 g of fish meat extract (trade name: Ehrlich Meat Extract; manufactured by Kyokuto Seiyaku Kogyo), 5 g of yeast extract (trade name; Meast P2G; manufactured by Asahi Food and Health Care), 20 g of glucose, 1 g of decaglycol monooleate (trade name: Polyglycerine Ester Poem J-0381V; manufactured by Riken Vitamin), 0.1 g of sodium acetate crystals (compliant with the Food Additive Standards), 0.1 g of magnesium sulfate crystals (compliant with the Food Additive Standards), and 2 g of dipotassium hydrogen phosphate (compliant with the Food Additive Standards), then pure water was added to make 1 L, then the mixture was adjusted to pH 6.8 with sodium hydroxide and sterilized in an autoclave at 121°C for 20 minutes to prepare Medium (1).

### Comparative Example 1

As a control, Media (2) to (7) were prepared as in Example 1 except that each of the following peptone was added in place of 10 g Casein Peptone Plus (trade name; manufactured by Organotechnie).
Medium (2): 10 g of wheat peptone (trade name: Wheat Peptone E1; manufactured by Organotechnie)
Medium (3): 10 g of soybean peptone (trade name: Soybean Peptone A2; manufactured by Organotechnie)
Medium (4): 10 g of soybean peptone (trade name : Soybean Peptone A3; manufactured by Organotechnie)
Medium (5): 10 g of soybean peptone (trade name: Soybean Peptone AX; manufactured by Organotechnie)
Medium (6): 10 g of plant peptone (trade name: Plant Peptone E1; manufactured by Organotechnie)
Medium (7): 10 g of casein peptone (trade name: Tryptone N1; manufactured by Organotechnie)

### Test Example 1

### 1. Preparation of lactic acid bacteria

Pre-culture of *Lactobacillus acidophilus* L-92 strain (FERM BP-4981) was inoculated at 3.5% to each of the media (1) to (7) prepared in Example 1 and Comparative Example 1 and cultured at 35°C for 18 hours.

After completion, of cutivation, the culture medium was centrifuged and the collected cells were washed with sterilized water for three times and freeze-dried in a freeze-drying device (FDU-830 manufactured by Tokyo Rika Kiki) to prepare power of lactic acid bacteria. All of the lactic acid bacterial powder were recovered and weighed. The resulting lactic acid bacterial powder was suspended in sterilized water and sterilized at 100°C for 10 minutes to prepare a suspension of lactic acid bacteria. The anti-allergic activity was measured as indicated by IL-12 inducing activity by the following method.

### 2. Measurement of IL-12 inducing ability

### (1) Preparation of spleen cells

An OVA solution of 50 µg/0.1 mL (containing equivalent amount of Alum) was intraperitoneally injected into a BALB/c mouse (female) of six weeks age. The mouse was bred for ten days and killed by dislocation of cervical vertebra and spleen was extracted. The cells were suspended in RPMI 1640 medium (containing 10% FBS and 25 mM HEPES) and passed through 70 µm cell strainer (manufactured by Felcon) to prepare single cells. RPMI 1640 medium (containing 10% of FBS and 25 mM HEPES) was added to the cell suspension at the viable cell numbers of 5 × 10⁶/ml to provide a spleen cell suspension. The number of viable cells was measured by mixing a trypan blue solution with the cell suspension and counting the number of viable cells with an erythrocyte calculating disk.

### Reagents:

### 1) FBS (Fetal bovine serum)

Cryopreserved FBS was inactivated in a water bath at 56°C for 30 minutes and placed in a sterilizing container and stored by freezing.

### 2) PBS

NaCl (80 g), 29 g of Na₂HPO₄.12H₂O, 2 g of KCl and 2 g of KH₂PO₄ were dissolved in distilled water to make 1 L and sterilized in an autoclave.

### 3) RPMI 1640 medium

RPMI 1640 medium "Nissui" (2) (trade name; manufactured by Nissui Seiyaku) (10.2 g) was dissolved in 1L of distilled water, sterilized in steam at 121°C for 20 minutes and then 10% of NaHCO₃, 0.3g of L-glutamine, 100, 000 units of penicillin and 0.1 g of streptomycin (manufactured by Gibco) were aseptically added.

### (2) Co-culture of spleen cells with lactic acid bacterial powder (induction of IL-12)

In a 96-well flat-bottom plate (manufactured by Falcon), the spleen cell suspension (200 µL/well), 0.01 to 1 µg/well of lactic acid bacterial powder suspension and 20 µg/well of OVA were added and incubated at 37°C under 5% CO₂ for 24 hours.

### (3) Measurement of IL-12 as an indicator for anti-allergic activity

The supernatant of the culture was assayed for IL-12 using a mouse IL-12 p70 measuring kit (trade name: OptEIA Mouse IL-12 (p70) Set; manufactured by BD Phar Mingen) in a 96-well immunoassay plate (manufactured by Nunc). The result is shown in Fig. 1.

### (4) Result

Referring to Fig. 1, the medium (1) containing casein peptone plus showed a very high inducing ability for IL-12 production, while the media containing other peptone showed lower ability. The medium (1) containing casein peptone plus had the or even higher inducing ability for IL-12 production as compared with a commercially available MRS medium.

### Example 2

### Preparation of tablets

Casein peptone (trade name: Casein Peptone Plus; manufactured by Organotechnie) (1 kg), 0.5 kg of yeast extract (trade name: Yeast Peptone Standard Type F; manufactured by Organotechnie), 2.25 kg of sucrose, 0.1 kg of decaglycerol monooleate (trade name: Sunsoft Q-17S; manufactured by Taiyo Kagaku), 0.5 kg of sodium acetate crystals (compliant with the Food Additive Standards), 0.5 kg of dipotassium hydrogen phosphate (compliant with the Food Additive Standards), 0.1 kg of magnesium sulfate crystals (compliant with the Food Additive Standards) and 1 kg of fish meat extract (trade name: Bacterio-N-KS(B); manufactured by Maruha) were mixed and messed-up to 100 L by addition of pure water. The mixture was adjusted to pH 6.8 with 50% sodium hydroxide (compliant with the Food Additive Standards), sterilized at 121°C for 20 minutes according to a conventional method, and cooled to the culture temperature of 35°C to prepare a medium.

A starter culture prepared at 35°C was aseptically inoculated at 1 to 5% into the medium and stirred for 15 minutes, then continued cultivation without stirring until pH reached 4.2 or lower (for about 18 hours). After completion of the cultivation, the culture medium was centrifuged with type SC-1 centrifuge (manufactured by Westphalia) to separate into insoluble solid and liquid phase. The cells were washed by suspending the resulting insoluble solid in pure water to the same volume as the original culture medium and separating with type SC-1 centrifuge (manufactured by Westphalia) into insoluble solid and liquid phase. The resulting insoluble solid was suspended in pure water to an extent of the same volume as the original culture medium, sterilized by heating at 100°C for 10 minutes, cooled to 60°C or lower and centrifuged with type SC-1 centrifuge (manufactured by Westphalia) to separate into insoluble solid and liquid phase. In this way, a washed, sterilized and concentrated cell dispersion was prepared.

The cell dispersion was placed into a freeze-drying vat to a thickness of 1 cm, and frozen in a freezer at -38°C until the dispersion temperature reached -35°C or lower. The frozen dispersion of the cells was quickly placed in a freeze-drying machine TFD-50LF2 (manufactured by Toyo Giken) and dried by a conventional method with the initial heating temperature of 40°C until the Loss of Drying value of the resulting dry product became 3% or less. The resulting dry product was pulverized by a Nara's free mill type M-4 (manufactured by Nara Kikai) and sieved through a 22 mesh screen to provide bacterial powder with uniform particle size.

The resulting bacterial powder (3.2 kg) was mixed with 55 of Pertech SI 150 (manufactured by Showa Kosan), 20 kg of powdery reduced palatinose PNP (manufactured by Mitsui Bussan), 10 kg of pure dry sterilized tapioca (manufactured by Matsutani Kagaku), 9.8 kg of glucose (compliant with the Food Additive Standards) and 2 kg of DK ester F-20W (manufactured by San-Ei Gen FFI) in a V-shaped blender of type UV-1 (manufactured by Uchida Kogyosho) by a conventional method for 5 minutes to provide powder for formulation of tablets containing the cell powder. The powder was formulated into tablets of 350 mg according to the conventional method using a Pegasus tabletting machine type 1024HUK-AWC (manufactured by Kikusui Seisakusho) with a 9-mm standard mortar-pestle of type R.

By administering two pieces of the resulting tablets per day, it is possible to ingest lactic acid bacteria in the same amount as those contained in the fermented milk, which was subjected to a clinical test on patients suffering from hay fever and year-round allergic rhinitis. According to the method of the invention, an anti-allergic agent can be produced which is easy to ingest in a continuous manner and can be manufactured at low costs.

### Example 3

### Preparation of beverage

Casein peptone (trade name: Casein Peptone Plus; manufactured by Organotechnie) (1 kg), 0.5 kg of yeast extract (trade name: Yeast Peptone Standard Type F; manufactured by Organotechnie), 2.25 kg of sucrose, 0.1 kg of decaglycerol monooleate (trade name: Sunsoft Q-17S; manufactured by Taiyo Kagaku), 0.5 kg of sodium acetate crystals (compliant with the Food Additive Standards), 0.5 kg of dipotassium hydrogen phosphate (compliant with the Food Additive Standards), 0.1 kg of magnesium sulfate crystals (compliant with the Food Additive Standards) and 1 kg of fish meat extract (trade name: Bacterio-N-KS(B); manufactured by Maruha) were mixed and messed-up to 100 L by addition of pure water. The mixture was adjusted to pH 6.8 with 50% sodium hydroxide, sterilized at 121°C for 20 minutes according to a conventional method and cooled to the culture temperature of 35°C to prepare a medium.

A starter culture prepared at 35°C was aseptically inoculated at 1 to 5% to the medium and stirred for 15 minutes, then continued cultivation without stirring until OD₆₀₀ reached 5.0 (for about 18 hours). After completion of the cultivation, the culture medium was centrifuged with type SC-1 centrifuge (manufactured by Westphalia) to separate into insoluble solid and liquid phase. The cells were washed by suspending the resulting insoluble solid in pure water to an extent of the same volume as the original culture medium and separating with type SC-1 centrifuge (manufactured by Westphalia) into insoluble solid and liquid phase. The resulting insoluble solid was suspended in pure water to an extent of the same volume as the original culture medium, sterilized by heating at 105°C for 3 minutes, cooled to 60°C or lower and centrifuged with type SC-1 centrifuge (manufactured by Westphalia) into insoluble solid and liquid phase. In this way, a washed, sterilized and concentrated cell dispersion was prepared.

The cell dispersion was placed into a freeze-drying vat to a thickness of 1 cm, and frozen in a freezer at -38°C until the dispersion temperature reached -35°C or lower. The frozen dispersion of the cells was quickly placed in a freeze-drying machine TFD-50LF2 (manufactured by Toyo Giken) and dried by a conventional method with the initial heating temperature of 40°C until the Loss on Drying value of the resulting dry product became 3% or less. The resulting dry product was pulverized by a Nara's free mill type M-4 (manufactured by Nara Kikai) and sieved through a 22 mesh screen to provide bacterial powder with uniform particle size.

The resulting bacterial powder (0.2 kg) was added to 1 ton of lactic acid bacterial beverage, which is a milk product with SNF of about 3% prepared by a conventional method, and homogenized at 15 MPa in a variable high-pressure homogenizer of type H-50 (manufactured by Sanwa Kikai). The resulting liquid material was sterilized at 96°C for 30 seconds by a conventional method using a plate heater for the manufacture of beverage and filled in 100-ml bottles at 85°C. PP cap was applied by winding and screwing according to a conventional method and turned upside down to sterilize the cap part to prepare a sterilized lactic acid bacterial beverage product.

By taking one bollte of the resulting lactic acid bacterial beverage product per day, it is possible to ingest lactic acid bacteria in the same amount as those contained in the fermented milk, which was subjected to a clinical test on patients suffering from hay fever and year-round allergic rhinitis. According to the method of the invention, a beverage product can be produced which is easy to ingest in a continuous manner and can be manufactured at low costs.

## Claims

1. A method for preparing a lactic acid bacterium having an anti-allergic activity, comprising culturing the lactic acid bacterium in a medium containing casein hydrolysate.

2. A method for preparing a lactic acid bacterium having an activity of stimulating the production of IL-12 comprising culturing the lactic acid bacterium in a medium containing casein hydrolysate.

3. The method according to claim 1 or 2, wherein the casein hydrolysate is pancreatin hydrolysate.

4. The method according to any of claims 1 to 3, wherein the hydrolysate is peptone.

5. The method according to any of claims 1 to 4, wherein the medium contains between 0.01 and 10% by mass of casein hydrolysate.

6. The method according to any of claims 1 to 5, wherein the lactic acid bacterium is a lactic acid bacterium belonging to genus *Lactobacillus*.

7. The method according to claim 6, wherein the lactic acid bacterium belonging to genus *Lactobacillus* is of *Lactobacillus acidophilus, Lactobacillus fermentum, Lactobacillus casei, Lactobacillus* paracasei or *Lactobacillus gasseri.*

8. The method according to claim 7, wherein the bacterium of *Lactobacillus acidophilus* is of *Lactobacillus acidophilus* L-92 strain (FERM BP-4981).

9. The method according to any of claims 1 to 8, wherein cells of the lactic acid bacterium is collected from the culture and dried.

10. The method according to any of claims 1 to 9, wherein the medium contains casein hydrolysate, and a nitrogen source derived from farmed meat or fish meat.

11. An anti-allergic agent comprising as an effective ingredient the lactic acid bacterium prepared by the method according to any of claims 1 to 10.

12. A food/beverage product having an anti-allergic activity comprising the lactic acid bacterium prepared by the method according to any of claims 1 to 10 as an effective ingredient.

13. An agent for stimulating production of IL-12 comprising as an effective ingredient the lactic acid bacterium prepared by the method according to any of claims 1 to 10.
